(19) Europäisches Patentamt
European Patent Office
Office européen des brevets

(11) **EP 1 012 131 B1**

(12) # FASCICULE DE BREVET EUROPEEN

(45) Date de publication et mention de la délivrance du brevet:
**19.03.2003 Bulletin 2003/12**

(21) Numéro de dépôt: **98939795.5**

(22) Date de dépôt: **03.09.1998**

(51) Int Cl.[7]: **C07C 29/143**

(86) Numéro de dépôt international:
**PCT/IB98/01378**

(87) Numéro de publication internationale:
**WO 99/012877 (18.03.1999 Gazette 1999/11)**

(54) **REDUCTION ENANTIOSELECTIVE DE CETONES AVEC UN SYSTEME AGENT SILANIQUE/COMPOSE METALLIQUE/LIGAND CHIRAL**

ENATIOSELEKTIVE REDUKTION VON KETONEN MIT EINEM SYSTEM AUS SILANMITTEL / METALLISCHER VERBINDUNG / CHIRALEM LIGAND

ENANTIOSELECTIVE REDUCTION OF KETONES WITH A SILANE AGENT/METAL COMPOUND/CHIRAL LIGAND SYSTEM

(84) Etats contractants désignés:
**CH DE ES FR GB IT LI NL**

(30) Priorité: **09.09.1997 CH 211097**
**01.04.1998 CH 77898**

(43) Date de publication de la demande:
**28.06.2000 Bulletin 2000/26**

(73) Titulaire: **FIRMENICH SA**
**1211 Genève 8 (CH)**

(72) Inventeur: **MIMOUN, Hubert**
**F-01630 Challex (FR)**

(74) Mandataire:
**Salvaterra-Garcia, Maria de Lurdes et al**
**Firmenich SA**
**Département des Brevets**
**Case Postale 239**
**1211 Genève 8 (CH)**

(56) Documents cités:
**WO-A-96/12694**

- **M.B. CARTER, ET AL.: "Enantioselective hydrosilation of ketones with a chiral titanocene catalyst" JOURNAL OF THE AMERICAN CHEMICAL SOCIETY, vol. 116, no. 26, 28 décembre 1994, pages 11667-11670, XP002083957 WASHINGTON, DC, US cité dans la demande**
- **S. NITZSCHE, ET AL.: "Reduktionen mit Methyl-wasserstoff-polysiloxanen" ANGEWANDTE CHEMIE, vol. 69, no. 3, 1957, page 96 XP002083958 WEINHEIM, DE**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 012 131 B1

## Description

La présente invention a trait au domaine de la synthèse organique. Plus précisément, elle concerne un procédé pour la réduction asymétrique de cétones prochirales en alcools chiraux en utilisant, comme agent réducteur, un agent silanique, de préférence le polyméthylhydrosiloxane (PMHS) et des composés de zinc, cobalt ou cadmium, accompagnés de ligands choisis dans le groupe constitué par les amines, les imines, les alcools et les aminoalcools chiraux.

La réduction énantiosélective de cétones en alcools est un domaine sur lequel on trouve une activité scientifique prononcée, en raison de l'importance industrielle potentielle de cette réaction. La production d'alcools chiraux est d'une grande importance notamment dans l'industrie de la chimie fine, par exemple dans l'industrie pharmaceutique, dans l'industrie des parfums et des arômes, dans l'agrochimie et pour la production d'insecticides. On est à la recherche de procédés qui donnent des rendements et excès énantiomériques élevés, tout en utilisant des catalyseurs métalliques et des ligands facilement disponibles à des prix modérés, ces derniers critères s'appliquant aussi aux agents réducteurs.

Dans ce contexte, il convient de citer les publications de Buchwald et al., dont, en particulier, le brevet US 5,227,538 qui décrit la réduction énantiosélective de cétones à l'aide de silanes en présence de catalyseurs choisis parmi les composés des métaux appartenant aux groupes 3, 4, 5 et 6 du tableau périodique, ainsi que parmi les lanthanides et actinides, et plus particulièrement parmi les dérivés du titane, et utilisés en présence d'additifs chiraux tels que les amines, diamines et diols. Ces systèmes sont généralement peu actifs et conduisent à des excès énantiomériques modestes, généralement inférieurs à 40%.

Carter, Schiott, Gutierrez et Buchwald ont également décrit dans J. Am. Chem. Soc., 1994, 116, 11667 la réduction énantiosélective de cétones à l'aide de PMHS en présence de titanocènes chiraux activés par du butyllithium, mais ces systèmes présentent l'inconvénient d'être très coûteux et nécessitent des quantités relativement importantes, de l'ordre de 5%, de catalyseur par rapport au substrat.

Il convient encore de citer dans le contexte de la présente invention la demande de brevet internationale WO 96/12694 de la demanderesse qui décrit la réduction d'aldéhydes, cétones, esters et lactones à l'aide d'un système réducteur comprenant un dérivé silanique et un hydrure métallique, formé à partir d'un sel ou complexe métallique et d'un agent réducteur. Outre des sels de zinc, on peut utiliser des sels de cadmium, de manganèse et de fer en tant que précurseurs pour engendrer l'hydrure métallique. Selon un mode d'exécution préféré, le polyméthylhydrosiloxane (PMHS) est utilisé en tant que dérivé silanique. Ce procédé n'est cependant pas adapté à la production d'alcools chiraux.

## Exposé de l'invention

Nous avons maintenant découvert qu'il était possible de préparer économiquement des alcools secondaires chiraux de grande pureté optique par réduction de cétones prochirales au moyen d'un dérivé silanique, de préférence le PMHS, et en présence de dérivés de zinc, cobalt ou cadmium, complexés par des ligands chiraux tels que des aminoalcools, alcools, amines ou imines.

Le procédé comprend les étapes suivantes :

a) la réaction d'une cétone prochirale avec une quantité efficace d'un agent silanique, en présence d'un catalyseur dérivé d'un composé précurseur du zinc, cobalt ou cadmium et d'un ligand chiral choisi dans le groupe constitué par les amines, les imines, les alcools et les aminoalcools chiraux ;
b) l'hydrolyse du siloxane obtenu au moyen d'un agent approprié ;
c) la séparation et purification de l'alcool optiquement actif formé.

Le procédé est en particulier caractérisé par le fait que l'on peut utiliser des quantités faibles de composé précurseur du zinc, cobalt ou cadmium et de ligand. De surcroît, ces réactifs et catalyseurs sont peu coûteux et ne nécessitent pas de précautions particulières de manipulation, notamment en ce qui concerne la protection vis-à-vis de l'humidité et de l'air.

Sans vouloir préjuger du mécanisme de réaction du procédé de l'invention, il apparaît probable que la réaction qui caractérise ce procédé puisse être illustrée par le schéma suivant se référant à un mode d'exécution préféré :

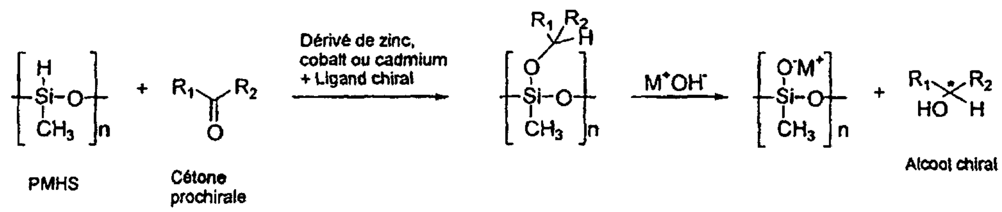

PMHS
Cétone prochirale
Alcool chiral

A titre d'agent silanique, on peut utiliser un dialkylsilane, un trialcoxysilane, un alkylchlorosilane ou un phénylsilane. On peut citer à titre d'exemple, le diméthylsilane, le diéthylsilane, le triméthoxysilane ou le triéthoxysilane. Selon un mode d'exécution préférentiel, on utilise en tant qu'agent silanique le PMHS ou polyméthylhydrosiloxane, qui se révèle très efficace et commercialement disponible. Par ailleurs, contrairement à d'autres silanes, le PMHS ne forme pas, dans une réaction de disproportionation, de silanes gazeux comme le $SiH_4$, gaz pyrophorique et lacrymogène qui nécessite des précautions particulières.

Les agents silaniques, qui servent en tant qu'agent réducteur dans l'invention, sont utilisés en quantité efficace pour que la conversion soit complète. En général, on utilisera une quantité au moins stochiométrique de l'agent silanique, et selon un mode d'exécution préféré de l'invention, on aura recours à un léger excès en agent silanique de l'ordre d'environ 10 à 40% par rapport à la quantité stochiométrique. Bien entendu, la réaction de réduction selon l'invention se déroule également lorsqu'on utilise l'agent silanique en quantités sous-stochiométriques, ceci entraînant, cependant, une baisse de conversion. Donc, par "quantité efficace" on veut dire ici une quantité suffisante d'agent silanique pour obtenir la réduction du substrat de façon industriellement efficace.

Le catalyseur selon l'invention peut être obtenu in situ, dans le milieu réactionnel, ou être préparé séparément. Dans tous les cas, le catalyseur est obtenu à partir d'un composé métallique précurseur et d'un ligand chiral choisi parmi le groupe des amines, des imines, des aminoalcools ou des alcools.

En tant que composé métallique précurseur, on peut utiliser des composés qui sont des dérivés de zinc, cobalt ou cadmium. Les composés métalliques préférés de la présente invention sont les composés de zinc, étant donné leur efficacité, leur manipulation facile et leur non-toxicité.

Dans un mode d'exécution de l'invention, on utilisera en tant que précurseur un composé qui est préparé in situ à partir d'un sel ou complexe de l'un des métaux cités auparavant et d'un agent réducteur. Ce mode d'exécution sera surtout utilisé lorsqu'on utilise des précurseurs métalliques instables ou sensibles, par exemple des espèces qui ne puissent être manipulées sans qu'une décomposition n'ait lieu. Par exemple, on peut préparer des hydrures métalliques en faisant réagir un sel ou complexe du métal respectif quelconque avec un agent réducteur tel que le $BH_3$ ou un borohydrure métallique de formule $M^+BH_4^-$ (M = Li, Na, K) ou $M(BH_4)_2$ (M=Mg, Ca, Zn), un alkylborane $MR_nBH_{(4-n)}$ (n=1 à 3, M=métal alcalin), un alcoxyborane $(RO)_nBH_{(4-n)}M$, (n=1 à 3, M=métal alcalin), un hydrure d'aluminium tel que $AlH_3$, $AlH_nR_{3-n}$, $MAlH_4$, $MAlH_nR_{4-n}$ ou $MAlH_n(OR)_{4-n}$ (M = Li, Na, K, n=1 à 3). Dans les formules données ci-dessus, R signifie un groupe alkyle, cycloalkyle, alcoxy, aryle, aryloxy, alcoxyalkyle, alcoxyaryle, aralcoxy, aralcoyle ou alkylaryle comprenant de 1 à 20 atomes de carbone. De préférence, R est un groupe alkyle de $C_1$ à $C_4$. On peut aussi préparer des dérivés organiques métalliques en faisant réagir un sel ou complexe de l'un des métaux cités auparavant avec un composé organolithien de formule LiR, organoaluminium de formule $AlR_3$ ou organo- gnésien de formule RMgX (X = Cl, Br, I), R ayant le sens défini ci-dessus. Des exemples spécifiques incluent le $NaBH_4$, le $LiAlH_4$ ou le $NaAlH_2(OCH_2CH_2OCH_3)_2$ (Vitride®). D'autres agents réducteurs que ceux cités auparavant peuvent être utilisés selon les connaissances générales dans l'art.

On peut utiliser pratiquement n'importe quel sel ou complexe du métal choisi dans la réaction avec l'agent réducteur pour engendrer le précurseur du catalyseur actif. A ce titre, il convient de citer en tant qu'exemples non limitatifs les halogénures (fluorures, chlorures, bromures, iodures), carbonates, cyanures, isocyanates, sulfates, phosphates, nitrates, carboxylates (acétates, propionates, 2-éthylhexanoates, stéarates, naphténates) ou alkoxides de zinc, cobalt ou cadmium.

En ne faisant référence qu'au métal préféré de l'invention, à savoir le zinc, on citera ici, comme exemples des composés précurseurs qui sont obtenus comme décrit ci-dessus à partir d'un sel ou complexe du zinc et d'un agent réducteur, l'hydrure de zinc de formule $ZnH_2$, un dérivé organique de formule générale $ZnR_2$, ou un hydrure organique de formule ZnHR, dans lesquelles R représente un groupe alkyle, cycloalkyle, alcoxy, aryle, aryloxy, alcoxyalkyle, alcoxyaryle, aralcoxy, aralcoyle ou alkylaryle comprenant de 1 à 20 atomes de carbone. De préférence, R est un groupe alkyle de $C_1$-$C_4$ dans ce mode d'exécution de la demande.

Nous avons également réussi à développer un autre mode d'exécution du procédé de la présente invention dans lequel on n'a pas recours à un composé précurseur qui est produit in situ par la réaction d'un sel ou complexe du métal choisi et d'un agent réducteur, mais on utilise directement, en tant que composé précurseur, un sel, complexe

ou un composé organique ou hydrique approprié du métal choisi. Après réaction avec le ligand chiral, le catalyseur actif est formé sans qu'une activation préalable par un agent réducteur soit nécessaire. Un agent réducteur peut quand même être utilisé après ou avant la réaction du ligand avec le composé précurseur, ce qui peut mener à des rendements ou excès énantiomériques accrus.

Les métaux que l'on emploie dans ce mode d'exécution sont les mêmes métaux que l'on a cité auparavant, c'est-à-dire le zinc, cadmium ou cobalt, le métal préféré étant le zinc. On utilisera alors un sel ou complexe de formule générale $MX_n$, dans laquelle M est choisi parmi les métaux mentionnés, X est un anion approprié et n est un nombre entier de 1 à 6. D'une façon surprenante, nous avons découvert que dans la réaction de réduction selon l'invention, il n'est pas nécessaire de faire emploi des espèces métalliques qui sont considérées comme étant hautement réactives, comme les hydrures ou alkyles qui sont engendrés comme décrit auparavant. Il s'est avéré qu'il existe un grand nombre d'anions X qui forment des sels ou complexes avec des métaux utiles dans la présente invention de la formule générale mentionnée $MX_n$, ces sels ou complexes étant capables de catalyser la réduction énantiosélective qui fait l'objet de l'invention.

De préférence, X est un anion choisi parmi un carboxylate, un alkoxyde de $C_1$-$C_{20}$, un β-dicétonate, un énolate, un amidure, un silylamide, un halogène, un carbonate ou un cyanure de zinc, cadmium ou cobalt. A ce titre, on utilisera préférentiellement les acétates, propionates, butyrates, isobutyrates, isovalérianates, diéthylacétates, benzoates, 2-éthylhexanoates, stéarates ou naphténates ; les méthoxydes, éthoxydes, isopropoxydes, tert-butoxydes, tert-pentoxydes ou 8-hydroxyquinolinates ; les acétylacétonates éventuellement substitués ou les tropolonates ; ou les fluorures.

Outre les sels ou complexes du zinc, cobalt et cadmium, il est aussi possible d'utiliser un composé organique ou hydrique de ces métaux. On utilisera un composé de formule $MX_n$ dans laquelle n est un nombre de 1 à 6 et X représente l'hydrogène ou un groupe organique tel qu'un groupe alkyle, cycloalkyle, aryle, alcoxyalkyle, alcoxyaryle, aralcoxy, aralcoyle ou alkylaryle comprenant de 1 à 20 atomes de carbone. De préférence, R est un groupe alkyle de $C_1$ à $C_4$ ou un hydrogène dans ce mode d'exécution. Il est aussi possible d'utiliser un composé mixte de formule $MX_n$ qui porte au moins un anion X tel que défini en haut pour les sels ou complexes de zinc, cadmium ou cobalt, et au moins un autre anion X constitué par un groupe organique ou un atome d'hydrogène.

En faisant référence au métal préféré de l'invention, à savoir le zinc, on utilisera, en tant que précurseur, un composé courant de formule générale $ZnX_2$ dans laquelle X a le sens indiqué ci-dessus. En ce qui concerne les composés du type alkylzinc, arylzinc ou hydrure de zinc, des exemples appropriés selon l'invention incluent le diméthylzinc, diéthylzinc, dipropylzinc, dibutylzinc, diphénylzinc, $ZnH_2$, ZnH(alkyle), ZnH(aryle), méthyl(méthoxy)zinc ou méthyl(phénoxy)zinc, ou un dérivé du type halogène(alkyl)zinc.

Comme mentionné auparavant, les ligands que l'on utilise dans le procédé de la présente invention appartiennent au groupe des amines, imines, alcools ou aminoalcools chiraux. Il s'est avéré que des diamines, diimines, diols, ainsi que des aminoalcools sont particulièrement appropriés pour utilisation dans la présente invention. Les ligands susmentionnés sont connus de l'homme de métier qui est à même de choisir le ligand dans ce groupe le plus avantageux pour la réduction d'une cétone donnée. En tant qu'exemples non limitatifs de ligands appropriés, on peut citer les familles de composés obéissant aux formules suivantes :

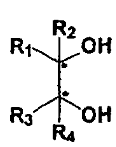
(I)

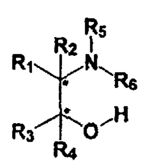
(II)

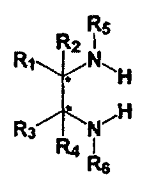
(III)

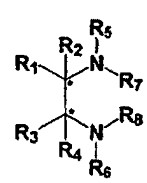
(IV)

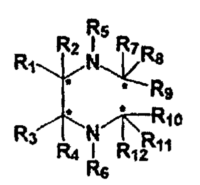
(V)

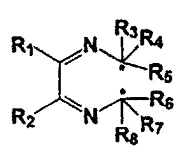
(VI)

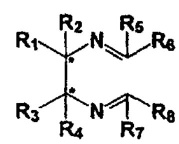
(VII)

Dans ces formules, les atomes de carbone notés d'un astérisque et/ou les groupes $R_1$ à $R_{12}$ peuvent représenter des centres stéréogéniques. Au moins l'un de ces centres stéréogéniques est à l'origine de la chiralité des ligands (I) à (VII). Les groupes $R_1$ à $R_{12}$ peuvent être chiraux ou non-chiraux et représentent des atomes d'hydrogène ou des substituants alkyle, cycloalkyle, alcoxy, aryle, aryloxy, alcoxyalkyle, alcoxyaryle, aralcoxy, aralcoyle ou alkylaryle comportant de 1 à 20 atomes de carbone.

Au cas où les atomes de carbone notés d'un astérisque ne représentent pas des centres stéréogéniques, la chiralité des ligands est le résultat de la chiralité d'au moins un des groupes R.

Par centre stéréogénique, on entend ici un atome qui donne lieu à une chiralité de la molécule, tel qu'un atome de carbone asymétrique ou un atome porteur d'une isomèrie atropique.

Selon un mode d'exécution préférentiel on utilisera un ligand selon l'une des formules (III) à (VII).

Le catalyseur actif de l'invention peut être décrit par la formule générale $ZnX_2L^*_n$, lorsque le zinc est utilisé. Dans cette formule, X est un anion, un groupe organique ou un hydrure, comme défini auparavant. L* représente un des ligands de formules (I) à (VII) cités plus haut et n représente un nombre entier de 1 à 6. Nous avons vérifié que le catalyseur actif de l'invention engendré par la réaction du composé précurseur avec le ligand L* respectif est une espèce monomérique du zinc, alors que les composés précurseurs sont, dans la plupart des cas, des espèces oligo- ou polymériques.

La chimie du zinc est en général caractérisée par le besoin que le métal a d'atteindre un nombre de coordination supérieur à 2, nombre qui lui est imposé par son état de valence +2. Par oligomérisation ou polymérisation, le zinc peut atteindre le nombre de coordination qu'il cherche à obtenir, en général la tétra- ou hexacoordination. Pour cette raison, les sels ou complexes de zinc sont le plus souvent oligo- ou polymériques. En tant qu'exemples, on mentionne ici les carboxylates et les halogénures de zinc.

Cependant, une classe de composés de zinc qui sont électroniquement insaturés sont les composés dialkyl- et les diarylzinc. Ils n'ont pas la possibilité d'oligomériser ou de polymériser puisque les groupes alkyles et aryles ne sont pas capables d'agir en tant que ligands pontants. Les composés dialkylzinc et diarylzinc sont, en conséquence, monomériques, et ils montrent une structure linéaire.

Nous avons pu vérifier que tous les composés mentionnés auparavant, c'est-à-dire les composés oligo- ou polymériques de zinc et les composés dialkyl- et diarylzinc, ne montrent aucune ou seulement une activité très faible s'ils sont utilisés pour la réduction (non-énantiosélective) de cétones. Cependant, ces espèces poly- ou oligomériques, ainsi que les composés de type dialkyl- ou diarylzinc, lorsque traités avec un ligand de formules (I) à (VII) qui engendre une espèce chirale monomérique, deviennent des catalyseurs hautement efficaces pour la réduction énantiosélective de cétones par un agent silanique selon l'invention.

Selon l'invention, on peut utiliser un sel ou complexe précurseur de zinc oligo- ou polymérique ou un composé dialkyl- ou diarylzinc et le transformer en catalyseur actif par traitement avec un agent complexant approprié. Nous avons par ailleurs constaté que l'on peut aussi utiliser des complexes ou sels de zinc monomériques connus, qui se sont révélés actifs dans le procédé de l'invention, alors qu'une telle activité est passée totalement inaperçue jusqu'à maintenant.

L'invention concerne également les complexes monomériques de zinc de formule générale $ZnX_2L^*_n$ dans laquelle X est un anion, un groupe organique ou un hydrure comme défini auparavant, L* est un ligand de formule (I) à (VII) et n est un nombre entier de 1 à 6. Cette classe de composés n'est pas connue de l'art antérieur. Des composés préférés sont ceux de la formule générale plus haut dans lesquels X est un carboxylate.

A titre préférentiel dans cette classe, on cite le Zn(diéthylacétate)$_2$[(S,S)-N,N'-éthylène-bis-(1-phényléthylamine)], le $Zn(CH_3)_2$[(S,S)-N,N'-éthylène-bis-(1-phényléthylamine)] et le $Zn(C_2H_5)_2$[(S,S)-N,N'-éthylène-bis-(1-phényléthylamine)]. La préparation et la caractérisation de ces composés est décrite plus loin.

Sans vouloir préjuger du mécanisme de la réaction, il est fort probable que l'espèce catalytique, dans le cas des autres métaux qui se prêtent à une utilisation dans la présente invention, est aussi un composé monomérique.

Par la mise en oeuvre du procédé selon la présente invention, il est possible d'obtenir des rendements de l'ordre de 100% et des excès énantiomériques (ee) proches de 90%. Le procédé de l'invention se révèle aussi très économique, compte-tenu de la disponibilité et des prix avantageux non seulement des agents silaniques, en particulier le PMHS, mais aussi des sels de zinc et des ligands employés, qui, en plus, sont utilisés en très faibles quantités, comme il ressort de ce qui suit.

La concentration en zinc par rapport au substrat est comprise entre 0,1 et 20% molaire et de préférence entre 0,5 et 5% molaire.

La concentration en ligand par rapport au zinc est comprise entre 5 et 200% molaire, de préférence entre 10 et 100% molaire.

Des concentrations similaires sont utilisées lorsqu'on emploie un composé du cadmium ou du cobalt.

Une particularité de la réaction selon l'invention, observée notamment lorsque le dérivé zincique est utilisé conjointement avec des ligands diimines ou diamines primaires et secondaires, est que le ligand peut être utilisé en quantité sous-stoechiométrique par rapport au métal, dans une quantité jusqu'à 10 fois inférieure, sans que le rendement ou l'excès énantiomérique n'en soient affectés. Cette particularité rend le procédé selon l'invention particulièrement attractif d'un point de vue économique, car le ligand représente le plus souvent la partie la plus onéreuse du système catalytique. Or, son utilisation selon l'invention à de très faibles concentrations, de l'ordre de 0,1 mole % par rapport au substrat, est très appréciable.

La réaction peut être effectuée à des températures très variées. A titre d'exemple on peut citer des températures de -20 à 100°C, et de préférence entre 0 et 60°C.

On peut opérer en l'absence de solvant ou en présence d'un solvant tel qu'un éther, par exemple le méthyl-tert-butyléther, le diisopropyléther, le dioxanne, le tétrahydrofuranne ou l'éthylèneglycol diméthyléther. On peut également opérer dans un solvant hydrocarboné tel que le cyclohexane, le toluène, l'heptane, l'octane, l'éther de pétrole, le xylène ou le mésitylène.

Le procédé selon l'invention peut être utilisé pour la réduction énantio- ou diastéréosélective de cétones.

A titre d'exemple non limitatif de ces cétones, on peut citer l'acétophenone et ses dérivés substitués, l'hexan-2-one, l'octan-2-one, la nonan-4-one, l'isophorone, la cyclohéxylméthylcétone, l'alpha-tétralone, la cyclohex-1-èn-2-one, la cyclopent-1-èn-2-one, la 2-pentyl cyclopent-1-èn-2-one, la beta-ionone, l'alpha-ionone, l'acetylfuranne, la dihydrobetaionone, les dicétones telles que la 2,5-hexanedione, les alpha et beta cétoesters, les cétolactones telles que la 2-cétopantolactone, les cétoamines, les cétoamides et les cétoacides.

## Manières de réaliser l'invention

Les complexes de type $ZnX_2L_n$ ont été préparés comme décrit ci-après.

**A.** Synthèse du complexe Zn(diéthylacétate)$_2$[(S,S,)-N,N'-éthylène-bis-(1-phényléthylamine)]

Ce composé a été préparé comme il est décrit ci-après, selon le schéma (1) :

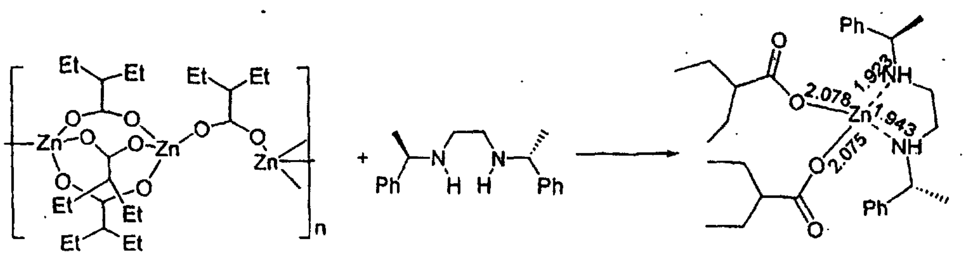
(1)

3 g (10 mmol) de diéthylacétate de zinc sont dissous dans 50 ml de diisopropyléther. On ajoute alors 3,1 g (10 mmol) de ligand (S,S,)-N,N'-éthylène-bis-( 1-phényléthylamine), et on agite à 20°C le mélange qui forme rapidement un précipité, qui est recueilli par filtration, puis recristallisé dans le cyclohexane. Rendement : 4,2 g (73%). L'analyse aux rayons X, effectuée à partir d'un monocristal, a permis de confirmer la structure de ce composé.

RMN($^1$H) : δ ppm : 0,95(12H, tt, $CH_3CH_2$) ; 1,55(6H, d, $CH_3$-CH) ; 1,6-1,7(8H, m, $CH_2$-CH) ; 2,3(m, 2H, CH-C=O) ; 2,55-2,8(m, 4H, $CH_2$-NH) ; 3,8(m, 2H, CH-NH) ; 7,2-7,4(m, 10H, arom) ;

RMN($^{13}$C) : δ ppm : 12,37 ; 12,42(q, $CH_3$) ; 23,36(q, $CH_3$) ; 25,99(t,$CH_2$-) ; 46,42(t, $CH_2$-NH) ; 51,15(d, CH-) ; 58,91 (d, CH-) ; 126-129(d,d,d) ; 141,19(s) ; 184,9(s, $CO_2$-).

**B**. Synthèse du complexe Zn($CH_3$)$_2$[(S,S,)-N,N'-éthylène-bis-(1-phényléthylamine)]

Ce composé a été préparé comme il est décrit ci-après, selon le schéma (2):

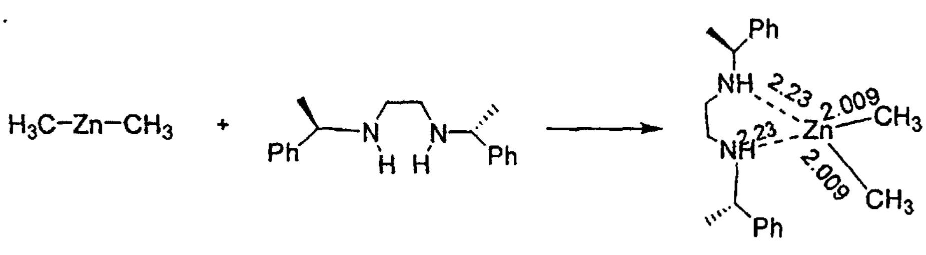
(2)

On mélange 9,7 mmol de diéthylzinc avec 2,5 g de N,N'-bis(1-(S)-phényléthyl)-1,2-éthylènediamine (9,33 mmol) dans 70 ml de toluène. Un précipité blanc se forme aussitôt. On évapore le toluène sous vide, le résidu est lavé au pentane et séché sous vide. On obtient ainsi 2,9 g de cristaux blancs (rendement 85%).
Analyse élémentaire : $C_{20}H_{30}N_2Zn$ : % poids calculé: C = 66,02; H = 8,31; N = 7,69. Trouvé : C = 66,7; H = 8,21; N = 7,73

RMN ($^1$H) ($CD_2Cl_2$, 25°C, δ ppm) : 7,07(m), 6,76(m), 3,54(m, 2H), 1,82(m, 2H), 1,63(m, 2H), 1,39(d, 6H, J=6,8Hz), 1,23(m,2H), -0,29(s,6H).

**C.** Synthèse du complexe $Zn(C_2H_5)_2$[(S,S,)-N,N'-éthylène-bis-(1-phényléthylamine)]

Ce composé a été préparé comme il est décrit ci-après, selon le schéma (3) :

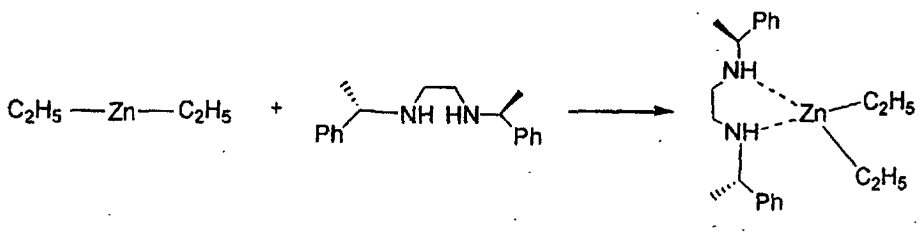
(3)

On mélange 1,40g de diéthylzinc (11,3 mmol) avec 3g de N,N'-bis(1-(S)-phényléthyl)-1,2-éthylènediamine (11,3 mmol) dans 50 ml de toluène et on laisse agiter 1 h à 20°C. On évapore le toluène sous vide, puis on ajoute 50 ml de pentane au résidu. La suspension est refroidie à -25°C pendant 1h, puis filtrée et séchée sous vide. On obtient ainsi 3,16 g de cristaux blancs (rendement 71%).
Analyse élémentaire : $C_{22}H_{34}N_2Zn$ : % poids calculé: C = 67,42; H = 8,74; N = 7,15. Trouvé : C = 67,51; H = 9,02; N = 7,06

IR (Nujol, $\nu_{max}$ $cm^{-1}$) : 3283 (m)
RMN ($^1$H) ($CD_2Cl_2$, 25°C, δ ppm) : 7,36-7,23 (m, 10H), 3,75 (m, 2H), 2,45 (m, 2H), 2,23 (m, 2H), 1,85 (m, 2H), 1,50 (d, 6H, J= 6,8 Hz), 1,3 (t, 6H, J=8,1 Hz), -0,04 (m, 4H).

L'invention est maintenant décrite plus en détail à l'aide des exemples suivants, dans lesquels les températures sont indiquées en degrés centigrades, les rendements en % molaires, et les excès énantiomériques (ee) par le rapport :

$$\%ee = 100 \times \frac{(R\text{-}S)}{(R+S)}$$

dans lequel (R) et (S) représentent les surfaces respectives des pics chromatographiques des deux énantiomères (R) et (S) en phase gazeuse sur colonne chirale de type Chirasil®.

Exemples 1 à 9

Réduction asymmétrique de l'acétophénone

Dans un ballon tricol de 100 ml, on introduit 10 g de toluène, puis 1 mmol de composé de zinc tel que le diéthylzinc (exemples 1 à 6, 9), l'hydrure de phénylzinc (exemple 7) ou l'hydrure de zinc (exemple 8), et 1 mmol de l'un des ligands de type diimine secondaire du tableau I présenté ci-après. On laisse agiter 10 min à 20°C, puis on introduit 12 g (100 mmole) d'acétophénone. On introduit alors en 10 minutes 6,5 g (100 mmole) de PMHS et on laisse la solution sous agitation pendant 8 h à 20°C. La disparition complète de l'acétophénone est suivie par chromatographie. On verse lentement la solution réactionnelle sur une solution aqueuse de soude à 30% (0,15 mole NaOH). On décante la phase aqueuse, on évapore le toluène de la solution organique, et on distille sous vide l'alcool résultant. L'excès énantiomérique du produit est déterminé par analyse chromatoraphique en phase gazeuse sur une colonne chirale de type Chirasil ®.
Les ligands diimines base de Schiff sont préparés par réaction de 1 équivalent de (1R,2R)-(-)diaminocyclohexane, disponible commercialement, avec 2 équivalents de 1-naphtaldéhyde (exemple 1), d'héliotropine (exemple 2), de mesitylaldéhyde (exemple 3), et de 2-formylpinane (exemple 4), suivant la description de Krasik et Alper, Tetrahedron, 1994, 50, p. 4347.

Les ligands diimines des exemples 5 à 8 sont obtenus par condensation entre deux équivalents de (R)-α-naphtyléthylamine (exemple 5) ou de (R)-(α)-phényléthylamine avec un équivalent de 2,3-butanedione suivant la description de tom Dieck et Dietrich, Chem. Ber., 1984, 117, p. 694.

Le ligand pyridinyl-imine de l'exemple 9 est préparé par condensation entre la 2-pyridyl carboxaldéhyde et la (R)-α-phényléthylamine selon la méthode de Brunner, Reiter et Riepl, Chem. Ber., 1984, 117, 1330.

TABLEAU I :

| Exemple | Composé de zinc | Ligand | Rendement en alcool (%) | % ee (configuration) |
|---|---|---|---|---|
| 1 | $ZnEt_2$ | 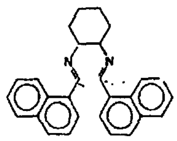 | 98 | 75 (S) |
| 2 | $ZnEt_2$ | 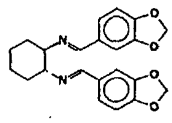 | 99 | 72 (S) |
| 3 | $ZnEt_2$ | 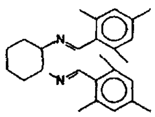 | 96 | 70 (S) |
| 4 | $ZnEt_2$ | 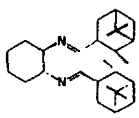 | 93 | 53 (S) |
| 5 | $ZnEt_2$ | 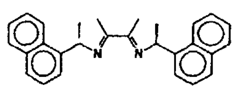 | 98 | 56 (S) |
| 6 | $ZnEt_2$ | 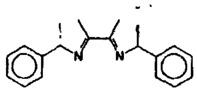 | 97 | 48 (S) |
| 7 | PhZnH.Py | 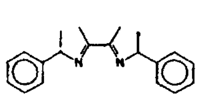 | 95 | 49 (S) |
| 8 | $ZnH_2$ | 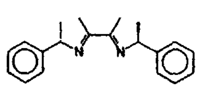 | 96 | 48 (S) |
| 9 | $ZnEt_2$ | 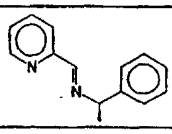 | 95 | 35 (S) |

Et = éthyle

Ph = phényle

Py = pyridine

Exemples 10 à 18

Réduction asymmétrique de l'acétophénone

On procède comme dans les exemples 1 à 9, en utilisant comme catalyseur le diéthylzinc (1 mmol) et comme ligand l'un des ligands diamines secondaires cités au tableau II ci-après (1 mmol). Après avoir agité le mélange à 20°C pendant 12 heures, on procède à l'hydrolyse à l'aide d'un excès de soude à 30%, et on récupère un mélange d'isomères (R) et (S) de l'alcool correspondant, présents dans les quantités indiquées au tableau II.
La N,N'-bis-(1-(S)-phényléthyl)-1,2-éthylènediamine (1 mmol), dont la formule est représentée dans l'exemple 11 du tableau II, a été préparée à partir de 1,2-dichloroéthane et de (S)-α-phényléthylamine selon la méthode de Hulst, de Vries et Feringa, Tetrahedron : Asymmetry, 1994, 5, 699.
Les autres diamines secondaires ont été préparées par réduction des diimines correspondantes à l'aide de borohydrure de sodium, dans un mélange toluène-méthanol (voir Corey, Jardine, Yuen et Connell, J. Am. Chem. Soc., 1989, 111, 9243) ou par l'hydrogène gazeux en présence de catalyseur oxyde de platine $PtO_2$ Adams (voir Alexakis, Mutti, et Mangeney, J. Org. Chem., 1992, 57, 1224).
Ces exemples montrent que les diamines secondaires chirales constituent des ligands particulièrement appropriés pour la réduction énantiosélective de l'acétophénone, conduisant à des rendements chimiques quasi-quantitatifs et des ee pouvant aller jusqu'à 88% (voir exemple 10).

TABLEAU II

| Exemple | Composé de zinc | Ligand | Rendement en alcool (%) | % ee (configuration) |
|---|---|---|---|---|
| 10 | $ZnEt_2$ | 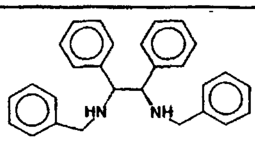 | 98 | 88 (S) |

| | | | | |
|---|---|---|---|---|
| 11 | $ZnEt_2$ | 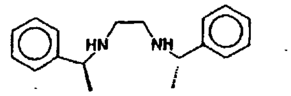 | 99 | 75 (R) |
| 12 | $ZnEt_2$ | 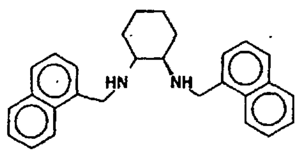 | 96 | 70 (S) |
| 13 | $ZnEt_2$ | 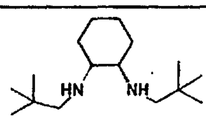 | 97 | 63 (S) |
| 14 | $ZnEt_2$ | 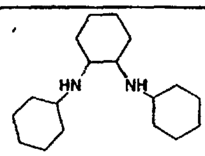 | 98 | 62 (S) |
| 15 | $ZnEt_2$ | 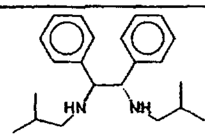 | 97 | 62 (S) |
| 16 | $ZnEt_2$ | 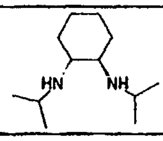 | 95 | 52 (S) |
| 17 | $ZnEt_2$ | 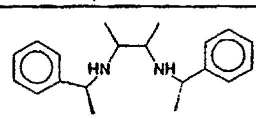 | 96 | 52 (S) |
| 18 | $ZnEt_2$ | 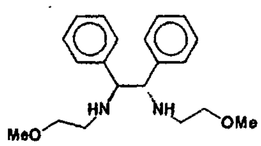 | 95 | 47 (R) |

Et = éthyle

Exemple 19

Réduction asymétrique de l'acétophénone

On procède comme dans l'exemple 11, en utilisant comme catalyseur le diéthylzinc (1 mmol) et comme ligand le N,N'-bis-(1-(S)-phényléthyl)-1,2-éthylènediamine que l'on utilise en quantité cinq fois inférieure, soit 0,2 mmol. Après avoir agité le mélange à 20°C pendant 24 heures, on procède à l'hydrolyse à l'aide d'un excès de soude à 30%, et on récupère avec un rendement de 95% un mélange de méthylphénylcarbinol composé de 88% d'isomère (S) et de 12% d'isomère (R), soit un ee de 74%.

Cet exemple montre que le ligand peut être employé en quantité sous-catalytique, soit 5 fois inférieure à celle de métal, sans que le rendement et l'excès énantiomérique n'en soient affectés.

Exemples 20 à 22

Réduction énantiosélective de l'acétophénone

On procède comme dans les exemples 1 à 9, mais en remplaçant le ligand par l'une des diamines tertiaires chirales du tableau III.
Le ligand de l'exemple 22 (R,R)-trans-l,2-bis(N-pyrrolidino)cyclohexane a été préparé suivant la description de Corey, Sarshar, Azimioara, Newbold et Noe, J. Am. Chem. Soc., 1996, 7851, à partir de (R,R)-diaminocyclohexane et de 1,4-dibromobutane en présence de triéthylamine. Le ligand de l'exemple 20 a été préparé similairement en remplaçant le dibromobutane par le α,α'-dichloro-o-xylène.
La sparteine utilisée dans l'exemple 21 est commercialement disponible.

TABLEAU III

| Exemple | Composé de zinc | Ligand | Rendement en alcool (%) | %ee (configuration) |
|---|---|---|---|---|
| 20 | $ZnEt_2$ | 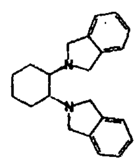 | 98 | 55 (S) |
| 21 | $ZnEt_2$ | 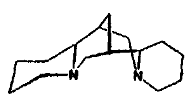 | 99 | 17 (S) |
| 22 | $ZnEt_2$ | 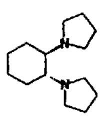 | 96 | 17 (S) |

Et = éthyle

Exemple 23

Réduction énantiosélective de l'acétophénone

On procède comme dans les exemples 1 à 9, en utilisant comme substrat l'acétophénone (100 mmol) et comme catalyseur le complexe isolé $Zn(C_2H_5)_2$[(S,S)-N,N'-éthylène-bis-(1-phényléthylamine)] (1 mmol). On laisse agiter le mélange à 20° pendant 18h, puis on procède à l'hydrolyse à l'aide d'un excès de soude à 30% et on récupère avec un rendement de 95% un mélange de méthylphénylcarbinol composé de 89% d'énantiomère (R) et 11% d'énantiomère (S), soit un ee de 78%.

Exemple 24

Réduction énantiosélective de l'acétophénone

On procède comme dans les exemples 1 à 9, en utilisant comme substrat l'acétophénone (100 mmol) et comme catalyseur le complexe isolé $Zn(diéthylacétate)_2$[(S,S)-N,N'-éthylène-bis-(1-phényléthylamine)] (1 mmol). On laisse agiter le mélange à 60° pendant 8h, puis on procède à l'hydrolyse à l'aide d'un excès de soude à 30% et on

récupère avec un rendement de 95% un mélange de méthylphénylcarbinol composé de 83% d'énantiomère (R) et 17% d'énantiomère (S), soit un ee de 65%.

Exemple 25

Réduction énantiosélective de l'acétophénone

Dans un ballon de 500 ml, on introduit 2,95g de diéthylacétate de zinc (10 mmol), 2,68g de (S,S)-N,N'- éthylène-bis-(1-phényléthylamine) (10 mmol) et l'on dissout le tout dans 100 ml de toluène pour préparer le complexe Zn-(diéthylacétate)$_2$[(S,S)-N,N'-éthylène-bis-(l-phényléthylamine)]. On ajoute alors à la solution de complexe 2g d'une solution de Vitride® $NaAlH_2(OCH_2CH_2OCH_3)_2$ à 70% dans le toluène (12 mmol). On observe un dégagement d'hydrogène et on agite 30 min. On introduit alors 120 g d'acétophénone (1 mol), puis 70 g de PMHS (1,08 mol) , et on laisse agiter 12 h à 20°C. Lorsque l'analyse GC indique que la consommation d'acétophénone est totale, on procède à l'hydrolyse à l'aide d'un excès de soude à 30%, et on récupère avec un rendement de 95% un mélange de méthylphénylcarbinol composé de 90% d'énantiomère (R) et 10% d'énantiomère (S), soit un ee de 80%.

Exemples 26 à 28

Réduction asymétrique de la 2-pentylcyclopent-1-èn-3-one

On procède comme dans les exemples 1 à 9, mais en prenant comme substrat la 2-pentylcyclopent-1-èn-3-one, et comme ligand utilisé en combinaison avec le diéthylzinc, l'un des aminoalcools chiraux commerciaux représentés dans le tableau IV. On obtient du 2-pentylcyclopent-1-èn-3-ol avec de bons rendements et avec les puretés optiques données dans le tableau IV.

TABLEAU IV

| Exemple | Composé de zinc | Ligand | Rendement en alcool (%) | %ee (Configuration) |
|---|---|---|---|---|
| 26 | $ZnEt_2$ | 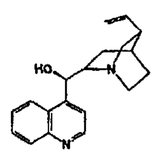 | 98 | 27 |
| 27 | $ZnEt_2$ | 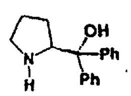 | 69 | 19 |
| 28 | $ZnEt_2$ | 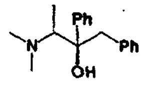 | 76 | 15 |

Et = éthyle

Ph = phényle

Exemples 29 à 36

Réduction énantiosélective de divers substrats

On procède comme dans les exemples 1 à 9, en prenant comme catalyseur le diéthylzinc (1 mol% par rapport au substrat) et le N,N'-bis-(1-(R)-phényléthyl)-1,2-éthylènediamine (1 mol%) mais en remplaçant l'acétophénone par l'une des cétones ou dicétones prochirales représentées dans le tableau V.
On obtient dans tous les cas majoritairement l'énantiomère (S) avec des ee compris entre 65 et 80%.

TABLEAU V

| Exemple | Substrat | Produit majoritaire | Rendement en alcool (%) | % ee (configuration) |
|---|---|---|---|---|
| 29 | 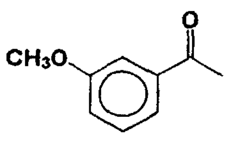 | 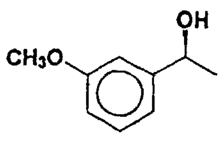 | 98 | 76 (S) |
| 30 | 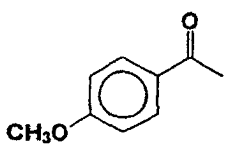 | 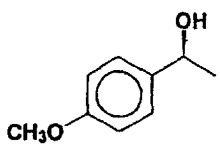 | 99 | 76 (S) |
| 31 | 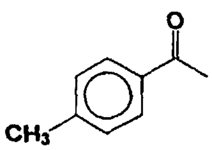 | 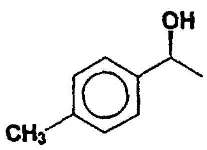 | 97 | 71 (S) |
| 32 | 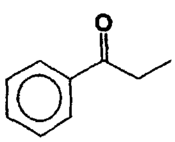 | 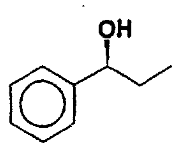 | 95 | 77 (S) |
| 33 | 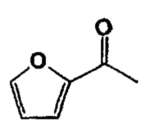 | 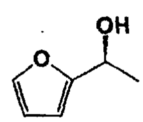 | 99 | 66 (S) |
| 34 | 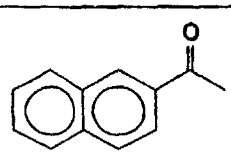 | 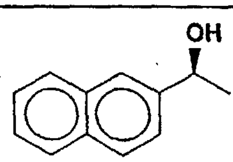 | 97 | 80 (S) |

| | | | | |
|---|---|---|---|---|
| 35 | 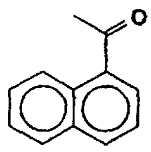 | 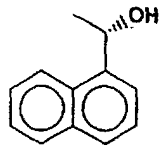 | 93 | 75 (S) |
| 36 | 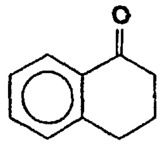 | 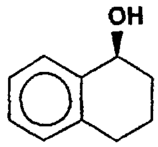 | 90 | 68 (S) |

<u>Exemple 37</u>

<u>Réduction énantiosélective de l'acétophénone avec un catalyseur de cadmium</u>

On procède comme dans les exemples 1 à 9, en utilisant comme substrat l'acétophénone (100 mmol), comme catalyseur le 2-éthylhexanoate de cadmium (1 mmol), et comme ligand la N,N'-bis(1-(S)-phényléthyl)-1,2-éthylènediamine (1 mmol) de l'exemple 11 du tableau II. On laisse agiter le mélange à 70°C pendant 8h, puis on procède à l'hydrolyse à l'aide d'un excès de soude à 30% et on récupère avec un rendement de 93% un mélange de méthylphénylcarbinol composé de 82% d'énantiomère (R) et 18% d'énantiomère (S), soit un ee de 64%.

<u>Exemple 38</u>

<u>Réduction énantiosélective de l'acétophénone avec un catalyseur de cadmium</u>

On procède comme dans les exemples 1 à 9, en utilisant comme substrat l'acétophénone (100 mmol), comme catalyseur le 2-éthylhexanoate de cadmium (1 mmol), et comme ligand la N,N'-dibenzyl (S,S)-diphényl 1,2-éthylènediamine (1 mmol) de l'exemple 10 du tableau II. On laisse agiter le mélange à 30°C pendant 18h, puis on procède à l'hydrolyse à l'aide d'un excès de soude à 30%. On récupère avec un rendement de 95% un mélange de méthylphénylcarbinol composé de 91% d'énantiomère (R) et 9% d'énantiomère (S), soit un ee de 82%.

<u>Exemple 39</u>

<u>Réduction énantiosélective de l'acétophénone avec un catalyseur de cobalt</u>

On procède comme dans les exemples 1 à 9, en utilisant comme substrat l'acétophénone (100 mmol), comme catalyseur le 2-éthylhexanoate de cobalt (1 mmol), et comme ligand la N,N'-bis(1-(S)-phényléthyl)-1,2-éthylènediamine (1 mmol) de l'exemple 11 du tableau II. On laisse agiter le mélange à 70°C pendant 8h, puis on procède à l'hydrolyse à l'aide d'un excès de soude à 30% et on récupère avec un rendement de 90% un mélange de méthylphénylcarbinol composé de 80% d'énantiomère (R) et 20% d'énantiomère (S), soit un ee de 60%.

**Revendications**

**1.** Procédé de réduction énantiosélective de cétones prochirales en alcools chiraux, ledit procédé comprenant les étapes suivantes:

a) la réaction d'une cétone prochirale avec une quantité efficace d'un agent silanique, en présence d'un catalyseur dérivé d'un composé précurseur de zinc, cobalt ou cadmium et d'un ligand chiral choisi dans le groupe constitué par les amines, les imines, les alcools et les aminoalcools chiraux ;
b) l'hydrolyse du siloxane obtenu au moyen d'un agent approprié ;
c) la séparation et purification de l'alcool optiquement actif formé.

**2.** Procédé selon la revendication 1, **caractérisé en ce que** le composé précurseur est préparé in situ par réaction d'un complexe ou d'un sel de zinc, cobalt ou cadmium avec un agent réducteur.

**3.** Procédé selon la revendication 2, **caractérisé en ce que** l'agent réducteur est:

a) un hydrure de bore de formule $BH_3$ ou $MBH_4$, M représentant Li, Na, ou K, ou de formule $M(BH_4)_2$, M représentant Mg, Ca ou Zn, un alkylborane de formule $MR_nBH_{(4-n)}$, ou un alkoxyborane de formule $(RO)_nBH_{(4-n)}M$, M représentant un métal alcalin, R un groupe alkyle, cycloalkyle, alcoxy, aryle, aryloxy, alcoxyalkyle, alcoxyaryle, aralcoxy, aralcoyle ou alkylaryle comprenant de 1 à 20 atomes de carbone et n un nombre de 1 à 3 ; ou
b) un hydrure d'aluminium de formule $AlH_3$, $AlH_nR_{3-n}$, $MAlH_4$, $MAlH_nR_{4-n}$ ou $MAlH_n(OR)_{4-n}$ dans laquelle M, R et n ont le sens indiqué ci-dessus ; ou
c) un agent répondant aux formules générales LiR, $AlR_3$ ou RMgX dans lesquelles X représente Cl, Br ou I et R a le sens indiqué ci-dessus.

**4.** Procédé selon la revendication 3, **caractérisé en ce que** l'agent réducteur est le $NaBH_4$, le $LiAlH_4$ ou le $NaAlH_2(OCH_2CH_2OCH_3)_2$.

**5.** Procédé selon l'une des revendications 1 à 4, **caractérisé en ce que** le composé précurseur est un composé de zinc.

**6.** Procédé selon l'une des revendications 2 à 5, **caractérisé en ce que** le complexe ou sel de zinc, cobalt ou cadmium est un fluorure, chlorure, bromure, iodure, carbonate, cyanure, isocyanate, sulfate, phosphate, nitrate, carboxylate ou un alkoxide de zinc, cobalt ou cadmium.

**7.** Procédé selon la revendication 6, **caractérisé en ce que** le complexe ou sel de zinc, cobalt ou cadmium est un acétate, propionate, 2-éthylhexanoate, stéarate ou naphténate.

**8.** Procédé selon la revendication 1, **caractérisé en ce que** le composé précurseur est un composé choisi parmi un carboxylate, un β-dicétonate, un énolate, un amidure, un silylamide, un halogénure, un carbonate, un cyanure, un hydrure, un groupe alkyle, cycloalkyle, alcoxy, aryle, aryloxy, alcoxyalkyle, alcoxyaryle, aralcoxy, aralcoyle ou alkylaryle comprenant de 1 à 20 atomes de carbone, de zinc, cobalt ou cadmium.

**9.** Procédé selon la revendication 8, **caractérisé en ce que** le composé précurseur est un composé de zinc.

**10.** Procédé selon la revendication 9, **caractérisé en ce que** le composé précurseur est un composé de type $ZnX_2$ dans laquelle X est un groupe acétate, propionate, butyrate, isobutyrate, isovalérianate, diéthylacétate, benzoate, 2-éthylhexanoate, stéarate, naphténate, méthoxyde, éthoxyde, isopropoxyde, tert-butoxyde, tert-pentoxyde, 8-hydroxyquinolinate ou acétylacétonate substitué ou non-substitué, tropolonate ou fluorure de zinc ou un composé de type $ZnH_2$, ZnHR, Zn(halogène)R ou $ZnR_2$ dans lesquelles R représente un groupe alkyle, cycloalkyle, alcoxy, aryle, aryloxy, alcoxyalkyle, alcoxyaryle, aralcoxy, aralcoyle ou alkylaryle comprenant de 1 à 20 atomes de carbone.

**11.** Procédé selon la revendication 10, **caractérisé en ce que** le composé de zinc est choisi parmi le diméthylzinc, diéthylzinc, dipropylzinc, dibutylzinc, diphénylzinc, méthyl(méthoxy)zinc ou méthyl(phénoxy)zinc.

**12.** Procédé selon l'une des revendications 1 à 11, **caractérisé en ce que** l'agent silanique est choisi dans le groupe constitué par les dialkylsilanes, les trialcoxysilanes, les alkylchlorosilanes et les phénylsilanes.

**13.** Procédé selon l'une des revendications 1 à 12, **caractérisé en ce que** l'agent silanique est le polyméthylhydrosiloxane (PMHS).

**14.** Procédé selon l'une des revendications 1 à 13, **caractérisé en ce que** le ligand chiral est choisi dans le groupe constitué par les diamines, les diimines et les aminoalcools chiraux.

**15.** Procédé selon la revendication 14, **caractérisé en ce que** le ligand chiral appartient à l'une des familles de composés représentées par les formules suivantes

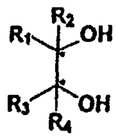
(I)

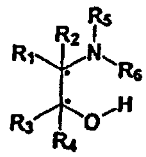
(II)

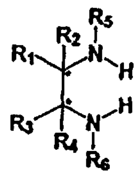
(III)

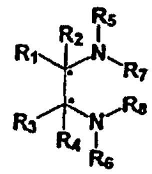
(IV)

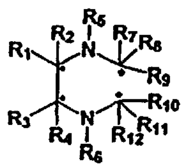
(V)

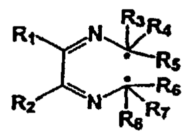
(VI)

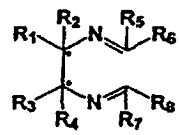
(VII)

dans lesquelles les centres stéréogéniques qui sont à l'origine de la chiralité des ligands se trouvent soit sur l'atome de carbone correspondant marqué d'un astérisque, soit sur les groupes $R_1$ à $R_{12}$, ces groupes $R_1$ à $R_{12}$ pouvant être chiraux ou non-chiraux et représentant des atomes d'hydrogène ou des substituants alkyle, cycloalkyle, alcoxy, aryle, aryloxy, alcoxyalkyle, alcoxyaryle, aralcoxy, aralcoyle ou alkylaryle comportant de 1 à 20 atomes de carbone.

**16.** Procédé selon la revendication 15, **caractérisé en ce que** le ligand chiral appartient à l'une des familles de composés représentés par l'une des formules (III) à (VII).

**17.** Procédé selon l'une des revendications 1 à 16, **caractérisé en ce que** la concentration du métal par rapport au substrat est comprise entre 0,1 et 20% molaire, et de préférence entre 0,5 et 5% molaire.

**18.** Procédé selon l'une des revendications 1 à 17, **caractérisé en ce que** la concentration du ligand par rapport au métal est comprise entre 5 et 200% molaire, et' de préférence entre 10 et 100% molaire.

**19.** Procédé selon la revendication 17 ou 18, **caractérisé en ce que** le métal est le zinc.

**20.** Complexe monomérique chiral de zinc, susceptible d'être obtenu par la réaction d'un composé précurseur mono-, oligo- ou polymérique de zinc et d'un ligand chiral.

**21.** Complexe monomérique selon la revendication 20, **caractérisé en ce que** le ligand est choisi parmi les ligands appartenant à l'une des familles de composés représentés par les formules suivantes

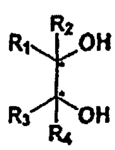
(I)

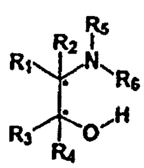
(II)

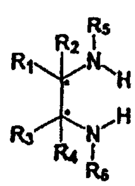
(III)

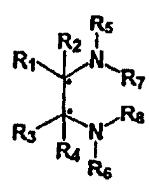
(IV)

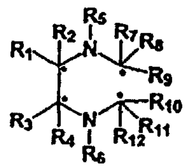
(V)

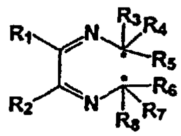
(VI)

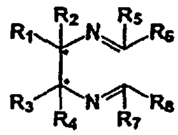
(VII)

dans lesquelles les centres stéréogéniques qui sont à l'origine de la chiralité des ligands se trouvent soit sur l'atome de carbone correspondant marqué d'un astérisque, soit sur les groupes $R_1$ à $R_{12}$, ces groupes $R_1$ à $R_{12}$ pouvant être chiraux ou non-chiraux et représentant des atomes d'hydrogène ou des substituants alkyle, cycloalkyle, alcoxy, aryle, aryloxy, alcoxyalkyle, alcoxyaryle, aralcoxy, aralcoyle ou alkylaryle comportant de 1 à 20 atomes de carbone.

**22.** Complexe selon la revendication 20 ou 21, **caractérisé en ce que** le composé précurseur est un sel, un complexe ou un composé organique ou hydrique de formule $ZnX_2$, dans laquelle X est un anion quelconque choisi parmi un carboxylate, un β-dicétonate, un énolate, un amidure, un silylamide, un halogénure, un carbonate, un cyanure ou un hydrure, ou un groupe alkyle, cycloalkyle, alcoxy, aryle, aryloxy, alcoxyalkyle, alcoxyaryle, aralcoxy, aralcoyle ou alkylaryle comprenant de 1 à 20 atomes de carbone.

**23.** Complexe selon la revendication 22, **caractérisé en ce que** X est un acétate, propionate, butyrate, isobutyrate, isovalérianate, diéthylacétate, benzoate, 2-éthylhexanoate, stéarate, naphténate, méthoxyde, éthoxyde, isopropoxyde, tert-butoxyde, tert-pentoxyde ou 8-hydroxyquinolate, un groupe acétylacétonate ou un dérivé supérieur de celui-ci, un groupe tropolonate, un atome de fluorure, un groupe méthyle, un groupe éthyle, un groupe propyle, un groupe butyle, un groupe phényle, un groupe méthoxy, un groupe phénoxy, ou un hydrure.

**24.** Complexe selon l'une des revendications 20 à 23, **caractérisé en ce qu'**il est représenté par la formule $ZnX_2L^*_n$, dans laquelle X est un anion tel que défini aux revendications 22 ou 23, L* est un ligand chiral tel que défini à la revendication 21, les ligands L* pouvant être identiques ou différents et le rapport ligand/zinc, exprimé par n, étant compris entre 1 et 6.

**25.** Un carboxylate monomérique et chiral de zinc.

**26.** En tant que carboxylate selon la revendication 25, le $Zn(\text{diéthylacétate})_2$ [(S,S)-N-N'-éthylène-bis-(1-phényléthylamine)].

**27.** $Zn(CH_3)_2$[(S,S)-N-N'-éthylène-bis-(1-phényléthylamine)].

**28.** $Zn(C_2H_5)_2$[(S,S)-N-N'-éthylène-bis-(1-phényléthylamine)].

**29.** Utilisation d'un complexe selon l'une des revendications 20 à 28 en tant que catalyseur pour la réduction énantiosélective de cétones avec un agent silanique.

## Patentansprüche

**1.** Verfahren für die enantioselektive Reduktion von prochiralen Ketonen zu chiralen Alkoholen, wobei das Verfahren die folgenden Schritte aufweist:

a) Umsetzung eines prochiralen Ketons mit einer wirksamen Menge eines Silan-Mittels in Gegenwart eines Katalysators, der von einer Zink-, Kobaltoder Cadmium-Vorläuferverbindung und von einem chiralen Liganden, der aus der Gruppe bestehend aus chiralen Aminen, Iminen, Alkoholen und Aminoalkoholen ausgewählt ist, abgeleitet ist;
b) Hydrolyse des erhaltenen Siloxans unter Verwendung eines geeigneten Mittels;
c) Abtrennung und Reinigung des gebildeten, optisch aktiven Alkohols.

**2.** Verfahren nach Anspruch 1, **dadurch gekennzeichnet, daß** die Vorläuferverbindung durch Umsetzung eines

Zink-, Kobalt- oder Cadmiumkomplexes oder -salzes mit einem Reduktionsmittel *in situ* bereitet wird.

**3.** Verfahren nach Anspruch 2, **dadurch gekennzeichnet, daß** das Reduktionsmittel ist:

a) ein Borhydrid der Formel $BH_3$ oder $MBH_4$, wobei M für Li, Na oder K steht, oder der Formel $M(BH_4)_2$, wobei M für Mg, Ca oder Zn steht, ein Alkylboran der Formel $MR_nBH_{(4-n)}$, oder ein Alkoxyboran der Formel $(RO)_nBH_{(4-n)}M$, wobei M für ein Alkalimetall, R für eine Alkyl-, Cycloalkyl-, Alkoxy-, Aryl-, Aryloxy-, Alkoxyalkyl-, Alkoxyaryl-, Aralkoxy-, Aralkyl- oder Alkylarylgruppe mit von 1 bis 20 Kohlenstoffatomen, und n für eine Zahl von 1 bis 3 steht; oder
b) ein Aluminiumhydrid der Formel $AlH_3$, $AlH_nR_{3-n}$, $MAlH_4$, $MAlH_nR_{4-n}$ oder $MAlH_n(OR)_{4-n}$, in der M, R und n die oben angegebene Bedeutung haben; oder
c) ein Mittel, das den allgemeinen Formeln LiR, $AlR_3$ oder RMgX entspricht, in der X für Cl, Br oder I steht, und R die oben angegebene Bedeutung hat.

**4.** Verfahren nach Anspruch 3, **dadurch gekennzeichnet, daß** das Reduktionsmittel $NaBH_4$, $LiAlH_4$ oder $NaAlH_2(OCH_2CH_2OCH_3)_2$ ist.

**5.** Verfahren nach einem der Ansprüche 1 bis 4, **dadurch gekennzeichnet, daß** die Vorläuferverbindung eine Zinkverbindung ist.

**6.** Verfahren nach einem der Ansprüche 2 bis 5, **dadurch gekennzeichnet, daß** der Komplex bzw. das Salz von Zink, Kobalt oder Cadmium ein Zink-, Kobalt- oder Cadmiumfluorid, -chlorid, -bromid, -iodid, -carbonat, -cyanid, -isocyanat, -sulfat, -phosphat, -nitrat -carboxylat oder ein Alkoxid von Zink, Kobalt oder Cadmium ist.

**7.** Verfahren nach Anspruch 6, **dadurch gekennzeichnet, daß** der Komplex bzw. das Salz von Zink, Kobalt oder Cadmium ein Acetat, Propionat, 2-Ethylhexanoat, Stearat oder Naphthenat ist.

**8.** Verfahren nach Anspruch 1, **dadurch gekennzeichnet, daß** die Vorläuferverbindung eine Verbindung ist, die aus einem Carboxylat, β-Diketonat, Enolat, Amidid, Silylamid, Halogenid, Carbonat, Cyanid, Hydrid, einer Alkyl-, Cycloalkyl-, Alkoxy-, Aryl-, Aryloxy-, Alkoxyalkyl-, Alkoxyaryl-, Aralkoxy-, Aralkyl- oder Alkylarylgruppe mit von 1 bis 20 Kohlenstoffatomen von Zink, Kobalt oder Cadmium ausgewählt ist.

**9.** Verfahren nach Anspruch 8, **dadurch gekennzeichnet, daß** die Vorläuferverbindung eine Zinkverbindung ist.

**10.** Verfahren nach Anspruch 9, **dadurch gekennzeichnet, daß** die Vorläuferverbindung eine Verbindung vom Typ $ZnX_2$ ist, wobei X eine substituierte oder nicht substituierte Acetat-, Propionat-, Butyrat-, Isobutyrat-, Isovalerat-, Diethylacetat-, Benzoat-, 2-Ethylhexanoat-, Stearat-, Naphthenat-, Methoxid-, Ethoxid-, Isopropoxid-, *tert*-Butoxid-, *tert*-Pentoxid-, 8-Hydroxychinolinat-, Acetylacetonatgruppe, Zinktropolonat oder -fluorid oder eine Verbindung vom Typ $ZnH_2$, ZnHR, Zn(Halogen)R oder $ZnR_2$ ist, wobei R für eine Alkyl-, Cycloalkyl-, Alkoxy-, Aryl-, Aryloxy-, Alkoxyalkyl-, Alkoxyaryl-, Aralkoxy-, Aralkyl- oder Alkylarylgruppe mit von 1 bis 20 Kohlenstoffatomen steht.

**11.** Verfahren nach Anspruch 10, **dadurch gekennzeichnet, daß** die Zinkverbindung aus Dimethylzink, Diethylzink, Dipropylzink, Dibutylzink, Diphenylzink, Methyl(methoxy)zink oder Methyl(phenoxy)zink ausgewählt ist.

**12.** Verfahren nach einem der Ansprüche 1 bis 11, **dadurch gekennzeichnet, daß** das Silan-Mittel aus der Gruppe bestehend aus Dialkylsilanen, Trialkoxysilanen, Alkylchlorsilanen und Phenylsilanen ausgewählt ist.

**13.** Verfahren nach einem der Ansprüche 1 bis 12, **dadurch gekennzeichnet, daß** das Silan-Mittel Polymethylhydrosiloxan (PMHS) ist.

**14.** Verfahren nach einem der Ansprüche 1 bis 13, **dadurch gekennzeichnet, daß** der chirale Ligand aus der Gruppe bestehend aus chiralen Diaminen, Diiminen und Aminoalkoholen ausgewählt ist.

**15.** Verfahren nach Anspruch 14, **dadurch gekennzeichnet, daß** der chirale Ligand zu einer der Familien von Verbindungen gehört, die durch die folgenden Formeln

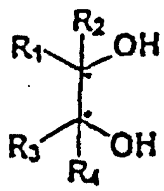

(I)

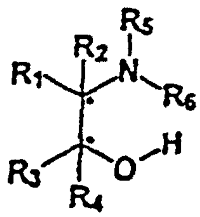

(II)

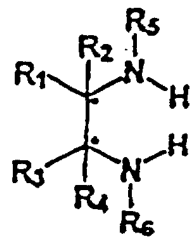

(III)

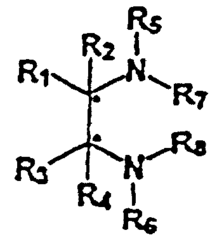

(IV)

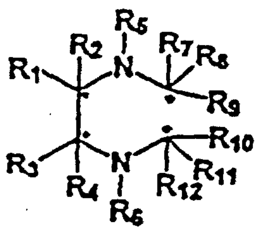

(V)

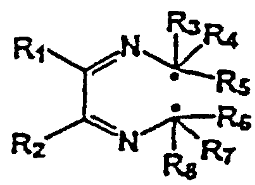

(VI)

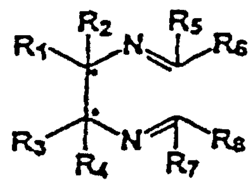

(VII)

dargestellt sind, in denen sich die für die Chiralität der Liganden ausschlaggebenden stereogenen Zentren entweder an dem ensprechenden, mit einem Asterisken gekennzeichneten Kohlenstoffatom oder an den Gruppen $R_1$ bis $R_{12}$ befinden, wobei diese Gruppen $R_1$ bis $R_{12}$ chiral oder achiral und Wasserstoffatome oder Alkyl-, Cycloalkyl-, Alkoxy-, Aryl-, Aryloxy-, Alkoxyalkyl-, Alkoxyaryl-, Aralkoxy-, Aralkyl- oder Alkylarylsubstituenten mit von 1 bis 20 Kohlenstoffatomen sein können.

**16.** Verfahren nach Anspruch 15, **dadurch gekennzeichnet, daß** der chirale Ligand zu einer der Familien von Verbindungen gehört, die durch eine der Formeln (III) bis (VII) dargestellt sind.

**17.** Verfahren nach einem der Ansprüche 1 bis 16, **dadurch gekennzeichnet, daß** die Konzentration des Metalls in bezug auf das Substrat zwischen 0,1 und 20 Mol-% und vorzugsweise zwischen 0,5 und 5 Mol-% beträgt.

**18.** Verfahren nach einem der Ansprüche 1 bis 17, **dadurch gekennzeichnet, daß** die Konzentration des Liganden in bezug auf das Metall zwischen 5 und 200 Mol-% und vorzugsweise zwischen 10 und 100 Mol-% beträgt.

**19.** Verfahren nach Anspruch 17 oder 18, **dadurch gekennzeichnet, daß** das Metall Zink ist.

**20.** Monomerer chiraler Zinkkomplex, der durch die Umsetzung einer mono-, oligo- oder polymeren Vorläufer-Zinkverbindung und eines chiralen Liganden erhältlich ist.

**21.** Monomerer Komplex nach Anspruch 20, **dadurch gekennzeichnet, daß** der Ligand unter den Liganden ausgewählt ist, die zu einer der Familie von Verbindungen gehört, welche durch die folgenden Formeln

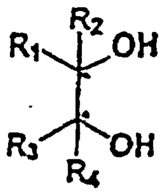
(I)

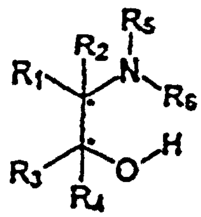
(II)

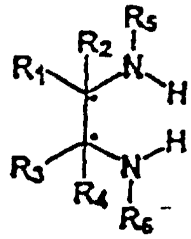
(III)

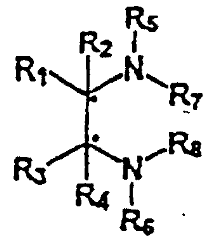
(IV)

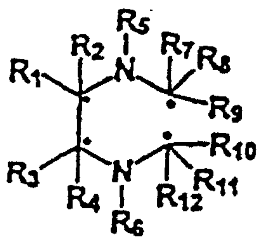
(V).

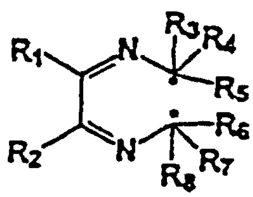
(VI)

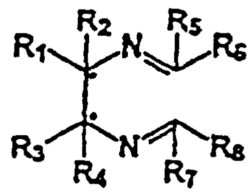
(VII)

dargestellt sind, in denen sich die für die Chiralität der Liganden ausschlaggebenden stereogenen Zentren entweder an dem ensprechenden, mit einem Asterisken gekennzeichneten Kohlenstoffatom oder an den Gruppen $R_1$ bis $R_{12}$ befinden, wobei diese Gruppen $R_1$ bis $R_{12}$ chiral oder achiral und Wasserstoffatome oder Alkyl-, Cycloalkyl-, Alkoxy-, Aryl-, Aryloxy-, Alkoxyalkyl-, Alkoxyaryl-, Aralkoxy-, Aralkyl- oder Alkylarylsubstituenten mit von 1 bis 20 Kohlenstoffatomen sein können.

**22.** Komplex nach Anspruch 20 oder 21, **dadurch gekennzeichnet, daß** die Vorläuferverbindung ein Salz, ein Komplex oder eine organische oder hydrierte Verbindung der Formel $ZnX_2$ ist, wobei X ein beliebiges Anion ist, das unter einem Carboxylat, β-Diketonat, Enolat, Amidid, Silylamid, Halogenid, Carbonat, Cyanid oder Hydrid, einer Alkyl-, Cycloalkyl-, Alkoxy-, Aryl-, Aryloxy-, Alkoxyalkyl-, Alkoxyaryl-, Aralkoxy-, Aralkyl- oder Alkylarylgruppe mit von 1 bis 20 Kohlenstoffatomen ausgewählt ist.

**23.** Komplex nach Anspruch 22, **dadurch gekennzeichnet, daß** X ein Acetat, Propionat, Butyrat, Isobutyrat, Isovalerat, Diethylacetat, Benzoat, 2-Ethylhexanoat, Stearat, Naphthenat, Methoxid, Ethoxid, Isopropoxid, *tert*-Butoxid, *tert*-Pentoxid oder 8-Hydroxychinolinat, eine Acetylacetonatgruppe oder ein höheres Derivat davon, eine Tropolonatgruppe, ein Fluoratom, eine Methyl-, Ethyl-, Propyl-, Butyl-, Phenyl-, Methoxy-, Phenoxygruppe oder ein Hydrid ist.

**24.** Komplex nach einem der Ansprüche 20 bis 23, **dadurch gekennzeichnet, daß** er durch die Formel $ZnX_2L^*_n$ dargestellt ist, wobei X ein Anion gemäß der Definition in den Ansprüchen 22 oder 23 ist, L* ein chiraler Ligand gemäß der Definition in Anspruch 21 ist, wobei die Liganden L* identisch oder voneinander verschieden sein können, und das durch n ausgedrückte Ligand/Zink-Verhältnis zwischen 1 und 6 liegt.

**25.** Monomeres und chirales Zinkcarboxylat.

**26.** Als Carboxylat nach Anspruch 25, Zn(Diethylacetat)$_2$-[(S,S)-N-N'-ethylen-bis-(1-phenylethylamin)].

**27.** Zn($CH_3$)$_2$[(S,S)-N-N'-Ethylen-bis-(1-phenylethylamin)].

**28.** Zn($C_2H_5$)$_2$[(S,S) -N-N'-Ethylen-bis-(1-phenylethylamin)].

29. Verwendung eines Komplexes nach einem der Ansprüche 20 bis 28 als Katalysator für die enantioselektive Reduktion von Ketonen mit einem Silan-Mittel.

## Claims

1. A process for the enantioselective reduction of prochiral ketones to chiral alcohols, said process comprising the following steps :

   a) the reaction of a prochiral ketone with an effective amount of a silane agent, in the presence of a catalyst derived from a zinc, cobalt or cadmium precursor compound and a chiral ligand selected from the group consisting of chiral amines, imines, alcohols and amino alcohols ;
   b) the hydrolysis of the siloxane obtained by using an appropriate agent;
   c) the separation and purification of the optically active alcohol formed.

2. A process according to claim 1, **characterised in that** the precursor compound is prepared in situ by reacting a zinc, cobalt or cadmium complex or salt with a reducing agent.

3. A process according to claim 2, **characterised in that** the reducing agent is:

   a) a boron hydride of formula $BH_3$ or $MBH_4$, M representing Li, Na, or K, or of formula $M(BH_4)_2$, M representing Mg, Ca or Zn, an alkylborane of formula $MR_nBH_{(4-n)}$, or a alkoxyborane of formula $(RO)_nBH_{(4-n)}M$, M representing an alkaline metal, R an alkyl, cycloalkyl, alkoxy, aryl, arylox, alkoxyalkyl, alkoxyaryl, aralkoxy, aralkoyl or alkylaryl group comprising from 1 to 20 carbon atoms and n a number from 1 to 3 ; or
   b) an aluminium hydride of formula $AlH_3$, $AlH_nR_{3-n}$, $MAlH_4$, $MAlH_nR_{4-n}$ or $MAlH_n(OR)_{4-n}$ wherein M, R and n have the meaning given above; or
   c) an agent corresponding to the general formulae LiR, $AlR_3$ or RMgX wherein X represents Cl, Br or I and R has the meaning given above.

4. A process according to claim 3, **characterised in that** the reducing agent is $NaBH_4$, $LiAlH_4$ or $NaAlH_2(OCH_2CH_2OCH_3)_2$.

5. A process according to any one of claims 1 to 4, **characterised in that** the precursor compound is a zinc compound.

6. A process according to any one of claims 2 to 5, **characterised in that** the zinc, cobalt or cadmium complex or salt is a zinc, cobalt or cadmium fluoride, chloride, bromide, iodide, carbonate, cyanide, isocyanide, sulphate, phosphate, nitrate, carboxylate or alkoxide.

7. A process according to claim 6, **characterised in that** the zinc, cobalt or cadmium complex or salt is an acetate, propionate, 2-ethylhexanoate, stearate or naphthenate.

8. A process according to claim 1, **characterised in that** the precursor compound is a compound selected from a zinc, cobalt or cadmium carboxylate, β-diketonate, enolate, amide, silylamide, halide, carbonate, cyanide, hydride, an alkyl, cycloalkyl, alkoxy, aryl, aryloxy, alkoxyalkyl, alkoxyaryl, aralkoxy, aralkoyl or alkylaryl group comprising from 1 to 20 carbon atoms.

9. A process according to claim 8, **characterised in that** the precursor compound is a zinc compound.

10. A process according to claim 9, **characterised in that** the precursor compound is a compound of the $ZnX_2$ type, wherein X is an acetate, propionate, butyrate, isobutyrate, isovalerate, diethyl acetate, benzoate, 2-ethyl hexanoate, stearate, naphthenate, methoxide, ethoxide, isopropoxide, tert-butoxide, tert-pentoxide, 8-hydroxyquinolinate or acetylacetonate group, substituted or non-substituted, a tropolonate or a zinc fluoride or a compound of the $ZnH_2$, ZnHR, Zn(halide)R or $ZnR_2$ type, wherein R represents an alkyl, cycloalkyl, alkoxy, aryl, aryloxy, alkoxyalkyl, alkoxyaryl, aralkoxy, aralkoyl or alkylaryl group comprising from 1 to 20 carbon atoms.

11. A process according to claim 10, **characterised in that** the zinc compound is selected from dimethylzinc, diethylzinc, dipropylzinc, dibutylzinc, diphenylzinc, methyl(methoxy)zinc or methyl(phenoxy)zinc.

**12.** A process according to any one of claims 1 to 11, **characterised in that** the silane agent is selected from the group consisting of dialkylsilanes, trialcoxysilanes, alkylchlorosilanes and phenylsilanes.

**13.** A process according to any one of claims 1 à 12, **characterised in that** the silane agent is polymethylhydrosiloxane (PMHS).

**14.** A process according to any one of claims 1 to 13, **characterised in that** the chiral ligand is selected from the group consisting of chiral diamines, diimines and aminoalcohols.

**15.** A process according to claim 14, **characterised in that** the chiral ligand belongs to one of the families of compounds represented by the following formulae

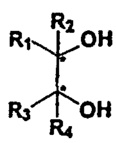
(I)

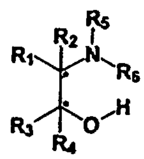
(II)

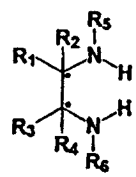
(III)

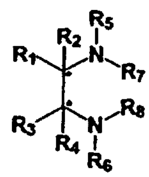
(IV)

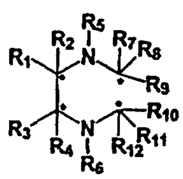
(V)

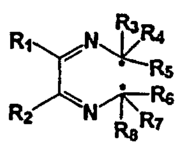
(VI)

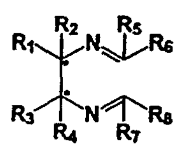
(VII)

wherein the stereogenic centres responsible for the chirality of the ligands are either at the corresponding carbon atom marked with an asterisk or at the groups $R_1$ to $R_{12}$, these groups $R_1$ to $R_{12}$ being optionally chiral or achiral and being hydrogen atoms or alkyl, cycloalkyl, alkoxy, aryl, aryloxy, alcoxyalkyl, alcoxyaryl, aralcoxy, aralcoyl or alkylaryl substituents comprising from 1 to 20 carbon atoms.

**16.** A process according to claim 15, **characterised in that** the chiral ligand belongs to one of the families of compounds represented by one of the formulae (III) to (VII).

**17.** A process according to any one of claim 1 to 16, **characterised in that** the concentration of the metal, based on the substrate, is comprised between 0,1 and 20 molar percent, and preferably between 0,5 et 5 molar percent.

**18.** A process according to any one of claims 1 to 17, **characterised in that** the concentration of the ligand, based on the metal, is comprised between 5 and 200 molar percent, and preferably between 10 and 100 molar percent.

**19.** A process according to claim 17 or 18, **characterised in that** the metal is zinc.

**20.** A monomeric chiral zinc complex, susceptible of being obtained by reacting a mono-, oligo- or polymeric precursor compound of zinc with a chiral ligand.

**21.** A complex according to claim 20, **characterised in that** the ligand is selected form the ligands belonging to one of the families of compounds represented by following formulae

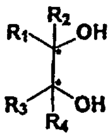

(I)

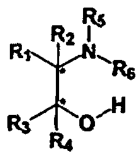

(II)

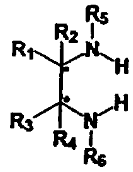

(III)

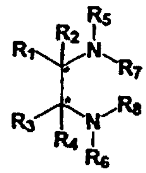

(IV)

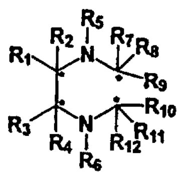

(V)

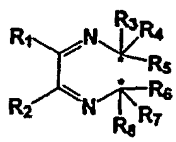

(VI)

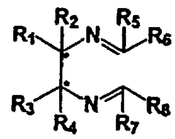

(VII)

wherein the stereogenic centres responsible for the chirality of the ligands are either at the corresponding carbon atom marked with an asterisk or at the groups $R_1$ to $R_{12}$, these groups $R_1$ to $R_{12}$ being optionally chiral or achiral and being hydrogen atoms or alkyl, cycloalkyl, alkoxy, aryl, aryloxy, alkoxyalkyl, alkoxyaryl, aralkoxy, aralkoyl or alkylaryl substituents comprising from 1 to 20 carbon atoms.

**22.** A complex according to claim 20 or 21, **characterised in that** the precursor compound is a salt, a complex or an organic or hydride compound of the formula $ZnX_2$, wherein X is any anion selected from a carboxylate, β-diketonate, enolate, amide, silylamide, halide, carbonate, cyanide or hydride, or an alkyl, cycloalkyl, alkoxy, aryl, aryloxy, alkoxyalkyl, alkoxyaryl, aralkoxy, aralkoyl or alkylaryl group comprising from 1 to 20 carbon atoms.

**23.** A complex according to claim 22, **characterised in that** X is an acetate, propionate, butyrate, isobutyrate, isovalerate, diethyl acetate, benzoate, 2-ethyl hexanoate, stearate, naphthenate, methoxide, ethoxide, isopropoxide, tert-butoxide, tert-pentoxide or 8-hydroxyquinolinate group, an acetyl acetonate group or a higher derivative thereof, a tropolonate group, a fluoride atom, a methyl group, an ethyl group, a propyl group, a butyl group, a phenyl group, a methoxy group, a phenoxy group or a hydride.

**24.** A complex according to any one of claims 20 to 23, **characterised in that** it is represented by the formula $ZnX_2L^*_n$, wherein X is an anion as defined in claim 22 or 23, L* is a chiral ligand as defined in claim 21, the ligands L* being the same or different, and the ligand/zinc ratio, expressed by n, being between 1 and 6.

**25.** A chiral monomeric zinc carboxylate.

**26.** As a carboxylate according to claim 25, $Zn(diethylacetate)_2$ [(S,S)-N-N'-ethylene-bis-(1-phenylethylamine)].

**27.** $Zn(CH_3)_2$[(S,S)- N-N'-ethylene-bis-(1-phenylethylamine)].

**28.** $Zn(C_2H_5)_2$[(S,S)- N-N'-ethylene-bis-(1-phenylethylamine)].

**29.** Use of a complex according to any one of claims 20 to 28 as a catalyst for the enantioselective reduction of ketones with a silane agent.